# EUROPEAN PATENT APPLICATION

(11) **EP 1 637 609 A1**
(43) Date of publication of application: **22.03.2006**
(21) Application number: 05013769.4
(22) Date of filing: 27.06.2005
(51) Int. Cl.: C12P 7/06

(54) **Equol-enriched plant extract obtainable by fermentation**

(30) Priority: 05.07.2004 IT MI20041342
(71) Applicant: MARFARMA HOLDING S.p.A., 20154 Milano (IT)
(72) Inventor: Heyda, Alessandro, 20154 Milano (IT)
(74) Representative: Bianchetti, Giuseppe

(57) **Abstract**

An equol-enriched plant extract obtainable through fermentation with *Eubacterium limosum* of isoflavones naturally contained in the extract is herein disclosed. The extract can be used in compositions useful for the treatment of the post-menopausal syndrome.

## Description

The present invention relates to an equol-enriched plant extract obtainable by treatment of conventional extracts with microorganisms able to transform isoflavones in equol.

The invention further relates to compositions containing said equol-enriched extracts for the treatment of the premenstrual syndrome and menopause-related symptoms, such as hot flashes, perspiration, headache and osteoporosis.

Soy or red clover isoflavones have been used for some time instead of estrogens in the treatment of the post-menopausal syndrome, since they show antioxidizing, antitumor, antinflammatory and cardioprotective activity.

The most abundant isoflavonoids are daizin and genistin, which are the glycoconjugates of daidzein and genistein.

Other isoflavones, present in particular in red clover extracts, comprise biochanin A, formonetin A and glycitein, characterized by the presence of methoxy groups on the phenyl rings.

Intestinal bacteria transform some of these isoflavones in equol, which proved the most active, as it is endowed with affinity towards both alpha and beta estrogen receptors (Setchell et al., Am. Soc. Nutritional Sciences, 2002,3577-3584).

Equol forms through daidzein demethylation, which can in turn derive from the metabolism of formonetin, whereas biochanin A can be converted to genistein.

Biochanina A, formonetin, genistein and daidzein are therefore some of the main sources of estrogenic activity in soy and clover extracts, which do not naturally contain equol.

It is also known that metabolic conversion of daidzein in equol takes place only in 30-40% of individuals, which causes various therapeutic responses in patients treated with soy or clover isoflavones extracts. Women that are able to convert natural isoflavones in equol show a lower risk of mammalian carcinoma.

Different combinations of soy and red clover extracts have been developed for a mutifactorial as possible treatment of the post-menopausal syndrome: for example, EP 1321149 (Marfarma) describes combinations of soy extracts and/or *Cimicifuga racemosa* with extracts of the so-called adaptogenic plants, which are able to provide relief from neurologic symptoms due to their properties, such as the nerve tonic, anxiolytic, antispasmodic and sedative activity.

US 2004106561 (Novogen Res.) discloses food supplements comprising phytoestrogens selected from genistein, daidzein, formonetin and/or biochanin A.

Red clover (*Trifolium pratense*) extracts and methods for the preparation thereof are disclosed, for example, in EP 1391208 (Cognis Iberia) and in EP 1174144 (Linnea SA). These extracts contain different genistein, daidzein, formonetin and biochanin A percentages.

In view of the above, it would be desirable to provide an equol-based composition, since it has been established that equol has a remarkable activity of on estrogen receptors; moreover, such a composition would allow to overcome the variability in the therapeutic response due the inability of most women (about two thirds of the population) to produce equol.

Hur et al. in FEMS Microbiol. Letters, 192 (2000), 21-25 reported that Eubacterium limosum (a fully anaerobic bacteria usually present in the human intestine), which is able to carry out O-demethylation of different substrates, is able to demethylate biochanin A, formonetin and glycitein to the corresponding demethyl-derivatives daidzein, genistein and 6,7,4'-trihydroxyisoflavones. Equol was not present also after prolonged incubation of the substrates with the culture broth.

It has now surprisingly been found that an equol-enriched red clover extract can be obtained by subjecting a clover extract to fermentation with Eubacterium limosum cells.

"Equol-enriched extract" means an extract containing from 0.05 to 15% by weight of equol on the dry extract.

Even if any Eubacterium limosum strain is suitable for carrying out the invention, in the following we will refer to strains from public collections, such as ATCC, DSMZ, Institut Pasteur, etc.. In particular, Eubacterium limosum ATCC 8486 can be used.

Any commercially available red clover extract can be used according to the invention. Preferably, the extract has an isoflavones content from 5 to 50%, more preferably of about 40% and a formonetin content from about 15 to 25%.

Eubacterium limosum ATCC 8486 is grown in culture medium Brain Heart Infusion (BHI) at 37°C under anaerobic conditions and in the presence of iron salts, preferably iron chloride (from 0.1 ng/ml to 5 ng/ml, preferably 0.5 ng/ml). The extract is added to the culture in concentrations ranging from 0.1 to 1 mg/ml and, after a time ranging from 1 to 3 days, the cells are filtered off and the extract is purified, for example by filtration from the cells and conventional purification, such as washings, column chromatography, centrifugation, extractions and the like. Equol concentration in the fermented extract is determined by reverse-phase HPLC using a mixture of acetonitrile and acetic acid as eluent, with a 1 ml/min flow rate.

Typically, the isoflavones content of the resulting extract is as follows:
Equol: from 0.05 to 15%;
Biochanin A: from 10 to 20%;
Formonetin: from 15 to 30%;
Genistein: from 0.1 to 2%;
Daidzein: from 0.1 to 15%.

The extract of the invention can conveniently replace isoflavones sources with phytoestrogenic activity in conventional compositions, formulated with known techniques, carriers and excipients.

Preferably, the compositions of the invention will contain from 10 to 500 mg of the fermented extract per oral dose unit. Examples of said formulations comprise soft- or hard- gelatin capsules, tablets, granulates in sachets, tablets or controlled- and/or prolonged- release capsules.

As stated above, the compositions of the invention can also contain other components, for example adaptogenic plants extracts (Griffonia simplicifolia, Hypericum perforatum, chamomilla, valerian, sage, ginseng, linden extract and the like). Griffonia extract is preferred due to its high 5-hydroxytriptophan content.

Besides the oral route, the compositions of the invention can also be conveniently administered through the transdermal or transmucosal route, using suitable formulations in the form of gels, bioadhesive gels, lotions (optionally supported on plasters), according to conventional techniques.

The following example illustrates the invention in greater detail.

### EXAMPLE

37 g of Brain heart Infusion (BHI-Fluka) are dissolved in 950 ml of distilled water and the solution is sterilized in autoclave for 15' at 121°C.

The red clover extract solution has the following composition:
- 0.2% red clover extract with a 20.2% content of formonetin, 18.8% biochanin A, 0.50% genistein, 0.60% daidzein;
- 1% Tween 20® ;
- 0.016% sodium citrate;
- 98.8% distilled water;
- lactic acid up to pH 7.5.

2 ml of this solution are added to 8 ml of BHI culture broth, then *Eubacterium limosum* ATCC 8486 cells and 0.2 ml of a ferric chloride solution (1 ng/ml) are added under stirring.

The fermentation is carried out for 26 days under anaerobic conditions.

After filtration and washing of the filter with water and acetonitrile, an extract with the following HPLC composition is obtained:

18% formonetin, 9.8% biochanin A, 0.50% genistein 9.50, 2.20% daidzein and 0.50 equol.

## Claims

1. Equol-enriched plant extract obtainable through fermentation with *Eubacterium limosum* strains.

2. Extract as claimed in claim 1, wherein the plant is red clover (*Trifolium pratense*)

3. Extract as claimed in claim 1, wherein the *Eubacterium limosum* strain is *Eubacterium limosum* ATCC 8486.

4. Extract as claimed in claim 1 containing from 0.05 to 15% equol by weight on the total dry extract.

5. Process for the preparation of the extract of claims 1-4, which comprises the addition of a red clover extract having a 5 to 50% isoflavones content to an anaerobic culture of *Eubacterium limosum* and subsequent purification of the extract.

6. Process as claimed in claim 5, wherein the *Eubacterium limosum* strain is *Eubacterium limosum* ATCC 8486.

7. Process as claimed in claim 5 or 6 wherein the culture medium is Brain Heart Infusion (BHI) in the presence of ferric salts, preferably ferric chloride (1 ng/ml).

8. Compositions containing the extract of claims 1-4 in admixture with suitable carriers.

9. Compositions as claimed in claim 8, suitable for the oral, transdermal or transmucosal administration.

10. Compositions as claimed in claim 8 suitable for the oral administration, in the form of soft- or hard- gelatin capsules, tablets, granulates in sachets, controlled-release and/or prolonged tablets or capsules.

11. Compositions as claimed in claim 8 suitable for the parenteral or transmucosal administration, in the form of gel, bioadhesive gel and lotions optionally supported on plasters.

12. Compositions as claimed in any one of claims from 8 to 11, further containing medicinal plant extracts.

13. Compositions as claimed in claim 12 wherein the medicinal plants are adaptogenic plants.

14. Compositions as claimed in claim 13, in which the adaptogenic plants comprise *Griffonia simplicifolia, Hypericum perforatum,* chamomilla, valerian, sage, ginseng and linden.

15. Compositions as claimed in claim 14, in which the adaptogenic plant is *Griffonia simplicifolia.*

16. Use of the extract of claims 1-4 for the preparation of compositions for the treatment of the post-menopausal syndrome.
